(19) Europäisches Patentamt
European Patent Office
Office européen des brevets

(11) Veröffentlichungsnummer : **0 246 190 B1**

(12) # EUROPÄISCHE PATENTSCHRIFT

(45) Veröffentlichungstag der Patentschrift : **04.12.91 Patentblatt 91/49**

(51) Int. Cl.[5] : **C07D 251/14**, C08K 5/34

(21) Anmeldenummer : **87810286.2**

(22) Anmeldetag : **06.05.87**

(54) **Isocyanursäurederivate.**

Die Akte enthält technische Angaben, die nach dem Eingang der Anmeldung eingereicht wurden und die nicht in dieser Patentschrift enthalten sind.

(30) Priorität : **12.05.86 CH 1918/86**

(43) Veröffentlichungstag der Anmeldung : **19.11.87 Patentblatt 87/47**

(45) Bekanntmachung des Hinweises auf die Patenterteilung : **04.12.91 Patentblatt 91/49**

(84) Benannte Vertragsstaaten : **AT BE CH DE ES FR GB IT LI NL SE**

(56) Entgegenhaltungen :
**DE-A- 2 445 307**
**US-A- 3 531 483**

(73) Patentinhaber : **CIBA-GEIGY AG**
**Klybeckstrasse 141**
**CH-4002 Basel (CH)**

(72) Erfinder : **Stegmann, Werner, Dr.**
**Unter der Sonnhalde 9**
**CH-4410 Liestal (CH)**
Erfinder : **Troxler, Eduard, Dr.**
**St. Albanring 220**
**CH-4052 Basel (CH)**
Erfinder : **Hofmann, Peter, Dr.**
**Lerchenstrasse 57**
**CH-4059 Basel (CH)**
Erfinder : **Meier, Hans-Rudolf, Dr.**
**Route du Confin 54**
**CH-1723 Marly (CH)**
Erfinder : **Dubs, Paul, Dr.**
**Route des Pralettes 25**
**CH-1723 Marly (CH)**

**EP 0 246 190 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft neue Cycloalkyl-p-hydroxybenzylisocyanurate, deren Verwendung zum Stabilisieren von organischem Material und das mit deren Hilfe gegen thermischen, oxidativen und/oder aktinischen Abbau stabilisierte organische Material.

p-Hydroxybenzylisocyanurate und deren Verwendung zum Stabilisieren von organischem Material sind beispielsweise aus den US-Patentschriften 3 531 483 und 3 669 962 und der deutschen Offenlegungsschrift 2 445 307 bekannt.

Gegenstand der Erfindung sind Verbindungen der Formel I,

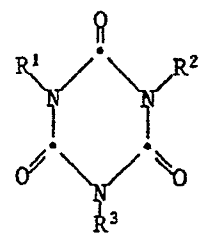

(I)

worin $R^1$ eine Gruppe der Formel II, $R^2$ eine Gruppe der Formel III und $R^3$ eine Gruppe der Formel IV bedeuten,

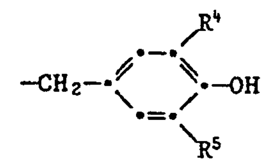

(II)

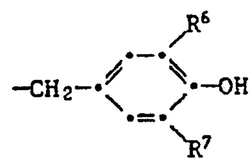

(III)

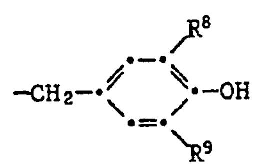

(IV)

worin $R^4$ $C_5$-$C_7$-Cycloalkyl oder durch $C_1$-$C_4$-Alkyl substituiertes $C_5$-$C_7$-Cycloalkyl ist, $R^5$, $R^6$, $R^7$, $R^8$ und $R^9$ unabhängig voneinander $C_1$-$C_{18}$-Alkyl, $C_5$-$C_7$-Cycloalkyl, durch $C_1$-$C_4$-Alkyl substituiertes $C_5$-$C_7$-Cycloalkyl, Phenyl, Benzyl oder Allyl sind.

Die Reste $R^4$ bis $R^9$ bedeuten als unsubstituiertes oder durch $C_1$-$C_4$-Alkyl substituiertes $C_5$-$C_7$-Cycloalkyl beispielsweise Cyclopentyl, Cyclohexyl, Cycloheptyl oder 1-Methylcyclohexyl. Bevorzugt ist Cyclohexyl.

Bedeuten die Reste $R^5$ bis $R^9$ $C_1$-$C_{18}$-Alkyl, so kann es sich um geradkettige oder verzweigte Reste handeln. Als Beispiele seien genannt: Methyl, Ethyl, Propyl, Isopropyl, n-Butyl, sec-Butyl, tert-Butyl, Pentyl, tert-Pentyl, Hexyl, Nonyl, Decyl, Dodecyl, Tetradecyl und Hexadecyl. Bevorzugt ist $C_1$-$C_4$-Alkyl, insbesondere Methyl, sec-Butyl und tert-Butyl.

Die Reste $R^1$, $R^2$ und $R^3$ können gleich oder verschieden sein.

Bevorzugt sind $R^1$, $R^2$ und $R^3$ gleich.

Besonders bevorzugt sind Verbindungen der Formel I, worin $R^1$ verschieden von $R^2$ ist und $R^2$ und $R^3$ gleich sind.

Ebenfalls bevorzugt sind Verbindungen der Formel I, worin $R^1$ und $R^2$ gleich sind und $R^2$ verschieden von $R^3$ ist.

Von Interesse sind Verbindungen der Formel I, worin $R^1$ eine Gruppe der Formel II, $R^2$ eine Gruppe der Formel III und $R^3$ eine Gruppe der Formel IV bedeuten, worin die Reste $R^5$ bis $R^9$ unabhängig voneinander

$C_1$-$C_8$-Alkyl, Cyclohexyl, 1-Methylcyclohexyl, Phenyl oder Allyl bedeuten.

Ebenfalls von Interesse sind Verbindungen der Formel I, worin $R^1$ eine Gruppe der Formel II, $R^2$ eine Gruppe der Formel III und $R^3$ eine Gruppe der Formel IV bedeuten, worin $R^4$ Cyclohexyl oder 1-Methylcyclohexyl ist. $R^4$ stellt bevorzugt Cyclohexyl dar.

Eine weitere Klasse bevorzugter Verbindungen der Formel I sind solche, in denen $R^1$ eine Gruppe der Formel II, $R^2$ eine Gruppe der Formel III und $R^3$ eine Gruppe der Formel IV bedeuten, worin $R^4$ Cyclohexyl und $R^5$ bis $R^9$ unabhängig voneinander Methyl, tert-Butyl, Cyclohexyl oder Phenyl darstellen.

Besonders bevorzugt sind Verbindungen der Formel I, worin $R^1$ eine Gruppe der Formel II, $R^2$ eine Gruppe der Formel III und $R^3$ eine Gruppe der Formel IV bedeuten, worin $R^4$, $R^6$ und $R^8$ Cyclohexyl sind und die Reste $R^5$, $R^7$ und $R^9$ gleich sind und Methyl, tert-Butyl, Cyclohexyl oder Phenyl darstellen.

Ebenfalls von Interesse sind Verbindungen der Formel I, worin $R^1$ eine Gruppe der Formel II, $R^2$ eine Gruppe der Formel III und $R^3$ eine Gruppe der Formel IV bedeuten, worin die Reste $R^5$, $R^7$ und $R^9$ gleich sind und Methyl oder Cyclohexyl bedeuten.

Von besonderem Interesse sind Verbindungen der Formel I, worin $R^1$ eine Gruppe der Formel II, $R^2$ eine Gruppe der Formel III und $R^3$ eine Gruppe der Formel IV bedeuten, worin $R^4$, $R^6$ und $R^8$ Cyclohexyl und $R^5$, $R^7$ und $R^9$ Methyl bedeuten, sowie solche Verbindungen, in denen die Reste $R^4$ bis $R^9$ $C_5$-$C_7$-Cycloalkyl oder Methylcyclohexyl, insbesondere Cyclohexyl, sind.

Beispiele für Verbindungen der Formel I sind:

N,N′,N″-Tris[3,5-dicyclohexyl-4-hydroxybenzyl]isocyanurat
N,N′,N″-Tris[3-cyclohexyl-5-methyl-4-hydroxybenzyl]isocyanurat
N,N′,N″-Tris[3-cyclohexyl-5-tert-butyl-4-hydroxybenzyl]isocyanurat N,N′-Bis[3,5-dicyclohexyl-4-hydroxybenzyl]-N″-(3,5-di-tert-butyl-4-hydroxybenzyl)isocyanurat
N,N′-Bis[3-cyclohexyl-5-methyl-4-hydroxybenzyl]-N″-(3,5-di-tert-butyl-4-hydroxybenzyl)isocyanurat
N,N′-Bis[3-cyclohexyl-5-tert-butyl-4-hydroxybenzyl]-N″-(3,5-di-tert-butyl-4-hydroxybenzyl)isocyanurat
N,N′-Bis[3,5-dicyclohexyl-4-hydroxybenzyl]-N″-(3-tert-butyl-5-methyl-4-hydroxybenzyl)isocyanurat
N,N′-Bis[3-cyclohexyl-5-methyl-4-hydroxybenzyl]-N″-(3-tert-butyl-5-methyl-4-hydroxybenzyl)isocyanurat
N,N′-Bis[3-cyclohexyl-5-tert-butyl-4-hydroxybenzyl]-N″-(3-tert-butyl-5-methyl-4-hydroxybenzyl)isocyanurat
N,N′-Bis[3,5-dicyclohexyl-4-hydroxybenzyl]-N″-(3,5-dimethyl-4-hydroxybenzyl)isocyanurat
N,N′-Bis[3-cyclohexyl-5-methyl-4-hydroxybenzyl]-N″-(3,5-dimethyl-4-hydroxybenzyl)isocyanurat
N,N′-Bis[3-cyclohexyl-5-tert-butyl-4-hydroxybenzyl]-N″-(3,5-dimethyl-4-hydroxybenzyl)isocyanurat
N,N′-Bis[3,5-di-tert-butyl-4-hydroxybenzyl]-N″-[3,5-dicyclohexyl-4-hydroxybenzyl]isocyanurat
N,N′-Bis[3,5-di-tert-butyl-4-hydroxybenzyl]-N″-[3-cyclohexyl-5-methyl-4-hydroxybenzyl]isocyanurat
N,N′-Bis[3,5-di-tert-butyl-4-hydroxybenzyl]-N″-[3-cyclohexyl-5-tert-butyl-4-hydroxybenzyl]isocyanurat
N,N′-Bis[3-tert-butyl-5-methyl-4-hydroxybenzyl]-N″-[3,5-dicyclohexyl-4-hydroxybenzyl]isocyanurat
N,N′-Bis[3-tert-butyl-5-methyl-4-hydroxybenzyl]-N″-[3-cyclohexyl-5-methyl-4-hydroxybenzyl]isocyanurat
N,N′-Bis[3-tert-butyl-5-methyl-4-hydroxybenzyl]-N″-[3-cyclohexyl-5-tert-butyl-4-hydroxybenzyl]isocyanurat
N,N′-Bis[3,5-dimethyl-4-hydroxybenzyl]-N″-[3,5-dicyclohexyl-4-hydroxybenzyllisocyanurat
N,N′-Bis[3,5-dimethyl-4-hydroxybenzyl]-N″-[3-cyclohexyl-5-methyl-4-hydroxybenzyl]isocyanurat
N,N′-Bis[3,5-dimethyl-4-hydroxybenzyl]-N″-[3-cyclohexyl-5-tert-butyl-4-hydroxybenzyl]isocyanurat

Besonders bevorzugte Verbindungen der Formel I sind:

N,N′,N″-Tris[3,5-dicyclohexyl-4-hydroxybenzyl]isocyanurat
N,N′,N″-Tris[3-cyclohexyl-5-methyl-4-hydroxybenzyl]isocyanurat
N,N′,N″-Tris[3-cyclohexyl-5-tert-butyl-4-hydroxybenzyl]isocyanurat
N,N′-Bis[3,5-dicyclohexyl-4-hydroxybenzyl]-N″-(3,5-di-tert-butyl-4-hydroxybenzyl)isocyanurat
N,N′-Bis[3,5-di-tert-butyl-4-hydroxybenzyl]-N″-[3,5-dicyclohexyl-4-hydroxybenzyl]isocyanurat
N,N′-Bis[3-cyclohexyl-5-methyl-4-hydroxybenzyl]-N″-(3,5-di-tert-butyl-4-hydroxybenzyl)isocyanurat
N,N′-Bis[3,5-di-tert-butyl-4-hydroxybenzyl]-N″-[3-cyclohexyl-5-methyl-4-hydroxybenzyl]isocyanurat

Die Verbindungen der Formel I können auf an sich bekannte Weise, z.B. wie in den US-Patentschriften 3 669 961 und 3 669 962 beschrieben, hergestellt werden, indem man ein Phenol der Formel V oder Mischungen von Phenolen der Formeln V, VI und VII

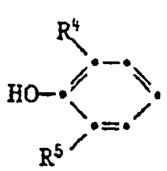 (V), 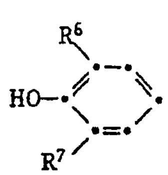 (VI), 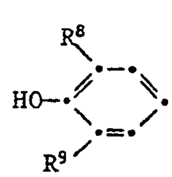 (VII),

worin die Reste $R^4$ bis $R^9$ die in den vorangehenden Ausführungen angegebenen Bedeutungen besitzen, mit Isocyanursäure und Formaldehyd oder einer Formaldehyd freisetzenden Verbindung umsetzt.

Bevorzugt werden die Reagentien in stöchiometrischem Verhältnis eingesetzt.

Es ist vorteilhaft, die Reaktion unter Stickstoff oder einem Edelgas, bevorzugt Argon, bei einer Temperatur zwischen 70° und 180°C durchzuführen. Eine Temperatur von ca. 130° bis 150°C ist bevorzugt.

Als Formaldehyd freisetzende Verbindungen eignen sich z.B. Formalinlösungen, Paraformaldehyd oder Trioxan.

Die Umsetzung kann ohne Katalysator oder in Gegenwart üblicher basischer Katalysatoren durchgeführt werden. Als Katalysatoren sind sekundäre und tertiäre Amine und Polyamine, wie beispielsweise Diethylamin, Tributylamin, Ethylendiamin, Tetramethylendiamin oder Hexamethylentetramin bevorzugt.

Die Reaktion erfolgt zweckmässigerweise in einem inerten organischen Lösungsmittel mit einem Siedepunkt, der oberhalb der Reaktionstemperatur liegt. Geeignete Lösungsmittel sind beispielsweise Formamid, Acetamid, Diethylformamid und bevorzugt Dimethylformamid oder Acetonitril.

Die Reaktion kann auch ohne Lösungsmittel oder in einem inerten Lösungsmittel oder Lösungsmittelgemisch mit einem Siedepunkt, der unterhalb der Reaktionstemperatur liegt, dann jedoch unter Druck, durchgeführt werden.

Es ist ausserdem vorteilhaft, wenn im Reaktionsmedium Wasser vorhanden ist.

Wenn Mischungen von Phenolen der Formeln V, VI und VII eingesetzt werden, fallen als Produkte Gemische von Isocyanuraten an, deren Trennung nach üblichen Methoden, beispielsweise durch Säulenchromatographie oder fraktionierte Kristallisation, erfolgen kann.

Die Ausgangsprodukte sind bekannt (teilweise im Handel erhältlich) und können nach bekannten Verfahren hergestellt werden. Die Herstellung von 2-Alkyl-6-cyclohexylphenolen und 2,6-Dicyclohexylphenolen wird beispielsweise in der US-Patentschrift 3 093 587 beschrieben.

Die Verbindungen der Formel I und deren Gemische eignen sich zum Stabilisieren von organischen Materialien gegen thermischen, oxidativen und/oder aktinischen Abbau. Beispiele für derartige Materialien sind:

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen- Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/ Ethylen- Acrylsäure-Copolymere, LLDPE/Ethylen-Vinylacetat-Copolymere und LLDPE/Ethylen-Acrylsäure-Copolymere.

3a. Kohlenwasserstoffharze (z.B. $C_5$-$C_9$) inklusive hydrierte Modifikationen davon (z.B. Klebrigmacherharze).

4. Polystyrol, Poly-(p-methylstyrol), Poly-(α-methylstyrol).

5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten

oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril- Alkylmethacrylat- Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten; Polyacetale, die mit termoplastischen Polyurethanen, Acrylaten oder MBS modifiziert sind.

13. Polyphenylenoxide und -sulfide und deren Mischungen mit Styrolpolymeren oder Polyamiden.

14. Polyurethene, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die mich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide susgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Vernetzte Polymere, die sich von Aldehyden einerseits und phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyestertharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

27. Mischungen (Polyblends) der vorgenannten Polymeren, wie z.B. PPE/PDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.
28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Minetalölen in beliebigen Gewichtsverhältnissen, wie sie z.B. als Spinnpräparationen Anwendung finden, sowie deren wässrige emulsionen.
29. Wässrige Emulsionen natürlicher oder synthetischer Kautschuke, wie z.B. Naturkautschuk-Latex oder Latices von carboxylierten Styrol-Butadien-Copolymeren.

Ein weiterer Gegenstand der Erfindung sind daher Zusammensetzungen, die organisches Material und mindestens eine Verbindung der Formel I enthalten.

Bei den organischen Materialien handelt es sich vorzugsweise um ein synthetisches Polymer, ein natürliches oder synthetisches Elastomer oder eine natürliche oder synthetische funktionelle Flüssigkeit, insbesondere ein gegen thermooxidativen oder/und lichtinduzierten Abbau empfindliches synthetisches Polymer. Polyolefine, wie z.B. Polypropylen und Polyethylen, sind besonders bevorzugte organische Materialien.

Als funktionelle Flüssigkeiten seien z.B. Hydraulikflüssigkeiten, Schmiermittel, Schmieröle usw. erwähnt.

Im allgemeinen werden die erfindungsgemässen Verbindungen dem zu stabilisierenden organischen Material in Mengen von 0,01 bis 10%, vorzugsweise 0,05 bis 5 %, insbesondere 0,05 bis 0,5 %, bezogen auf das Gesamtgewicht des zu stabilisierenden Materials, zugesetzt. Für die Stabilisierung können auch eventuell erhaltene Mischungen von Verbindungen der Formel I eingesetzt werden, ohne dass eine Trennung erfolgen muss. Dies trifft insbesondere bei solchen Verbindungen zu, worin $R_1$ bis $R_3$ nicht gleich sind und die aus dem Herstellungsverfahren in der Regel als Mischungen anfallen.

Ein weiterer Gegenstand dieser Erfindung sind daher physikalische Gemische enthaltend mindestens zwei Verbindungen der Formel I.

Diese Gemische können auch durch Vermischen der reinen Komponenten hergestellt werden.

Für die Stabilisierung von organischem Material kann es vorteilhaft sein, die erfindungsgemässen Verbindungen zusammen mit sogenannten Thiosynergisten einzusetzen. Thiosynergisten sind als sekundäre Antioxidantien oder Peroxidzerstörer bekannt und gehören u.a. zu den folgenden Substanzklassen:
Mercaptane, Thioether, Disulfide, Dithiocarbamate und heterocyclische Thioverbindungen. Beispiele für Thiosynergisten sind die folgenden Verbindungen:

Pentaerythrit-tetrakis[(β-alkylmercapto)propionat] wie beispielsweise Pentaerythrit-tetrakis[(β-dodecylmercapto)propionat]; Pentaerythrit-tetrakis(mercaptoacetat), 1,1,1-Trimethylolethantris(mercaptoacetat), 1,1,1-Trimethylolpropan-tris(mercaptoacetat), Dioleyl-3,3'-thiodipropionat, Dilauryl-3,3'-thiodipropionat, Ditridecyl-3,3'-thiodipropionat, Dimyristyl-3,3'-thiodipropionat, Distearyl-3,3'-thiodipropionat, Dicyclohexyl-3,3'-thiodipropionat, Dicetyl-3,3'-thiodipropionat, Dioctyl-3,3'-thiodipropionat, Dibenzyl-3,3'-thiodipropionat, Laurylmyristyl-3,3'-thiodipropionat, Diphenyl-3,3'-thiodipropionat, Di-p-methoxyphenyl-3,3'-thiodipropionat, Didecyl-3,3'-thiodipropionat, Dibenzyl-3,3'-thiodipropionat, Diethyl-3,3'-thiodipropionat, 3-Methylmercaptopropionsäurelaurylester, 3-Butylmercaptopropionsäurelaurylester, 3-Laurylmercaptopropionsäurelaurylester, 3-Octylmercaptopropionsäurephenylester, 3-Phenylmercaptopropionsäurelaurylester, 3-Benzylmercaptopropionsäurelaurylester, 3-(p-Methoxy)phenylmercaptopropionsäurelaurylester, 3-Cyclohexylmercaptopropionsäurelaurylester, 3-Hydroxymethylmercaptopropionsäurelaurylester, 3-Hydroxyethylmercaptopropionsäuremyristylester, 3-Methoxymethylmercaptopropionsäureoctylester, 3-Hydroxymethylmercaptopropionsäurelaurylester, 3-Hydroxyethylmercaptopropionsäuremyristylester, 3-Methoxymethylmercaptopropionsäureoctylester, 3-Carboxymethylmercaptopropionsäuredilaürylester, 3-Carboxypropylmercaptopropionsäuredilaurylester, Dilauryl-4,7-dithiasebacat, Dilauryl-4,7,8,11-tetrathiatetradecandioat, Dimyristyl-4,11-dithiatetradecandioat, Lauryl-3-benzthiazylmercaptopropionat;
Dialkyldisulfide, wie beispielsweise Dioctyldisulfid, Didodecyldisulfid, Dioctadecyldisulfid; Dialkylsulfide, wie z.B. Didodecylaulfid, Dioctadecylaulfid; Alkylthiopropionsäuren und deren Salze, wie beispielsweise 3-Laurylmercaptopropionsäure und dessen Calciumsalz oder die in den japanischen Offenlegungsschriften Sho 47-13533, Sho 47-24004, Sho 47-24541 und Sho 47-24005 beschriebenen schwefelhaltigen Verbindungen.

Bevorzugt werden die erfindungsgemässen Verbindungen zusammen mit den Lauryl- oder Stearylestern der β-Thio-dipropionsäure eingesetzt.

Die Erfindung betrifft daher auch organisches Material enthaltend mindestens eine Verbindung der Formel I und einen Thiosynergisten.

Das Gewichtsverhältnis von Thiosynergist zu erfindungsgemässem Stabilisator kann beispielsweise 1:1 bis 20:1, bevorzugt 2:1 bis 10:1 und besonders bevorzugt 3:1 bis 7:1 betragen.

Die stabilisierten Polymerzusammensetzungen der Erfindung können zusätzlich auch verschiedene herkömmliche Additive enthalten, wie beispielsweise:

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6- Tricyclohexylphenol, 2,6-Di-tert.butyl-4-methoxymethylphenol, 2,6-Di-nonyl-4-methylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.

1.3. Hydroxylierte Thiodiphenylether, z.B. 2,2′-Thio-bis-(6-tert.-butyl-4-methylphenol), 2,2′-Thio-bis-(4-octylphenol), 4,4′-Thio-bis-(6-tert.butyl-3-methylphenol), 4,4′-Thio-bis-(6-tert.butyl-2-methylphenol).

1.4. Alkyliden-Bisphenole, z.B. 2,2′-Methylen-bis-(6-tert.butyl-4-methylphenol), 2,2′-Methylen-bis-(6-tert.butyl-4-ethylphenol), 2,2′Methylen-bis-[4-methyl-6-(α-methylcyclohexyl)-phenol], 2,2′-Methylen-bis-(4-methyl-6-cyclohexylphenol), 2,2′-Methylen-bis-(6-nonyl-4-methylphenol), 2,2′-Methylen-bis- (4,6- di- tert.butylphenol), 2,2′-Ethyliden-bis-(4,6-di-tert.butylphenol), 2,2′-Ethyliden-bis-(6-tert.butyl-4-isobutylphenol), 2,2′-Methylen-bis-[6-(a-methylbenzyl)-4-nonylphenol], 2,2′-Methylen-bis-[6-(α,α-dimethylbenzyl)-4-nonylphenol], 4,4′-Methylen-bis-(2,6-di-tert.butylphenol), 4,4′-Methylen-bis-(6-tert.butyl-2-methylphenol), 1,1-Bis-(5-tert.-butyl-4-hydroxy-2-methylphenyl)-butan, 2,6-Bis-(3-tert.butyl-5-methyl-2-hydroxybenzyl)-4-methylphenol, 1,1,3-Tris-(5-tert.-butyl-4-hydroxy-2-methylphenyl)-butan, 1,1- Bis-(5-tert.butyl-4- hydroxy-2- methylphenyl)- 3- n-dodecylmercaptobutan, Ethylenglycol-bis-[3,3-bis-(3′-tert.butyl-4′-hydroxyphenyl)-butyrat], Bis-(3-tert.butyl-4-hydroxy-5-methylphenyl)-dicyclopentadien, Bis-[2-(3′-tert.butyl-2′-hydroxy-5′-methyl-benzyl)-6-tert.butyl-4-methyl-phenyl]-terephthalat.

1.5 Benzylverbindungen, z.B. 1,3,5-Tris-(3,5-di-tert.butyl-4-hydroxybenzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiol-terephthalat, 1,3,5-Tris-(3,5- di- tert.butyl- 4- hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.

1.6. Acylaminophenole, z.B. 4-Hydroxy-laurinsäureanilid, 4-Hydroxy-stearinsäureanilid, 2,4-Bis-(octylmercapto)-6-(3,5-di-tert.-butyl-4-hydroxyanilino)-s-triazin, N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxyethyl)-isocyanurat, N,N′-Bis-(hydroxyethyl)-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)-ethyl-isocyanurat, N,N′-Bis-(hydroxyethyl)-oxalsäurediamid.

1.9. Ester der β-(3,5-Dicyclohexyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N′-Bis-(hydroxyethyl)-oxalsäurediamid.

1.10. Amide der β-(3,5-Di-tert.butyl-4-hydroxyphenyl)-propionsäure, wie z.B. N,N′-Bis-(3,5-di-tert.butyl-4-hydroxyphenylpropionyl)-hexamethylendiamin, N,N′-Bis-(3,5-di-tert.butyl-4-hydroxyphenylpropionyl)-trimethylendiamin, N,N′-Bis-(3,5-di-tert.butyl-4-hydroxyphenylpropionyl)-hydrazin.

2. UV-Absorber und Lichtschutzmittel

2.1. 2-(2′-Hydroxyphenyl)-benztriazole, wie z.B. das 5′-Methyl-, 3′,5′-Di-tert.butyl-, 5′-tert.Butyl-, 5′-(1,1,3,3-Tetramethylbutyl)-, 5-Chlor-3′,5′-di-tert.butyl-, 5-Chlor-3′-tert.butyl-5′-methyl-, 3′-sec.Butyl-5′-tert.butyl, 4′-Octoxy-, 3′,5′-Di-tert.amyl-, 3′,5′-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2′,4′-Trihydroxy-, 2′-Hydroxy-4,4′-dimethoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.-butylphenylester,
3,5-Di-tert.butyl-4-hydroxybenzoesäurehexadecylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketonoxim, Nickelkomplexe des 1-Phenyl-4-lauroyl-5-hydroxypyrazols, gegebenenfalls mit zusätzlichen Liganden.

2.6. Sterisch gehinderte Amine, wie z.B. Bis-(2,2,6,6-tetramethylpiperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-l,2,3,4-butantetraoat, 1,1'-(1,2-Ethandiyl)-bis-(3,3,5,5-tetramethyl-piperazinon).

2.7. Oxalsäurediamide, wie z.B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o-und p-Methoxy- sowie von o- und p-Ethoxy-di-substituierten Oxaniliden.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrazin, 3-Salicyloylamino-1,2,4-triazol, Bis-(benzyliden)-oxalsäuredihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Das Gewichtsverhältnis von erfindungsgemässem Stabilisator zu herkömmlichem Additiv kann beispielsweise 1:0,5 bis 1:5 betragen.

Die Einarbeitung der Stabilisatorsubstanzen in das organische Material erfolgt nach bekannten Methoden. Die Zudosierung kann während jeder Verarbeitungsstufe vor der Formgebung erfolgen.

Die Erfindung betrifft auch die Verwendung von Verbindungen der Formel I zum Stabilisieren von organischem Material gegen thermischen, oxidativen und/oder aktinischen Abbau, wobei bevorzugte organische Materialien vorstehend angegeben sind.

Die folgenden Beispiele erläutern die Erfindung weiter. Alle Prozentangaben beziehen sich auf das Gewicht, soweit nicht anders angegeben.

Beispiel 1: Herstellung von N,N',N"-Tris[3,5-dicyclohexyl-4-hydroxybenzyl]isocyanurat

A) Bei Raumtemperatur werden in einer Apparatur bestehend aus einem Sulfierkolben (2,5 1) mit Propellerrührer, Innenthermometer, Rückflusskühler und einer Stickstoffbegasungsanlage

135,5 g (1,05 Mol) Cyanursäure,
853,5 g (3,3 Mol) 2,6-Dicyclohexylphenol,
114 g (3,8 Mol) Paraformaldehyd,
1,76 g (0,0126 Mol) Hexamethylentetramin,

1176 g (16 Mol) Dimethylformamid und
31,5 g (1,75 Mol) Wasser
vorgelegt. Die Suspension wird in einer Stickstoffatmosphäre unter gutem Rühren auf ca. 116°C geheizt und dann 24 Stunden am Rückfluss gekocht. Das Ende der Umsetzung gilt als erreicht, wenn weniger als 2 % 2,6-Dicyclohexylphenol im Reaktionsgemisch vorhanden sind (Bestimmung mittels Dünnschichtchromatographie).

Die inzwischen rote, klare Lösung wird auf Raumtemperatur abgekühlt und unter langsamem Rühren solange angeimpft, bis die Kristallisation einsetzt. Anschliessend wird die Suspension noch ca. 10 Stunden gerührt, dann innerhalb von 2 Stunden auf 0 bis 5°C abgekühlt und bei dieser Temperatur noch eine weitere Stunde gerührt.

Das Produkt wird mit einer Glasnutsche abfiltriert, mit 1000 g Methanol gewaschen und im Vakuumofen bei 40°C und 20 mbar getrocknet.

Die Ausbeute beträgt 610 g (65 % der Theorie).

Das Produkt besitzt einen Schmelzpunkt von 247°C.

Elementaranalyse:

Berechnet: C 76,6%; H 8,7 %; N 4,5 %; 0 10,2 %
Gefunden: C 76,7 %; H 8,8 %; N 4,7 %; 0 10,2 %

B) Die Apparatur besteht aus einem 500 ml Doppelmantelkolben mit Stahldeckel sowie Brüdenrohr mit Kugelhahn, Sicherheitsventil, Thermoelement und Druckaufnehmer.

In dem 500 ml Kolben werden nacheinander
38,7 g (0,3 mol) Cyanursäure,
244,1 g (0,946 mol) 2,6-Dicyclohexylphenol,
31,3 g (0,99 mol) 100%-iges (technisches) Paraformaldehyd,
0,5 g (0,0036 mol) Hexamethylentetramin,
150 g Dimethylformamid und
9 ml Wasser
vorgelegt. Unter Rühren wird bei Raumtemperatur auf 100 mbar evakuiert und mit Argon entlastet. Es wird wiederum auf 20 bis 30 mbar evakuiert und der Kolben via Kugelhahn geschlossen. Das Reaktionsgemisch wird nun bei verschlossenem Kolben innerhalb von 90 Minuten auf 130°C geheizt. Dabei geht die weisse Suspension bei ca. 120°C und einem Druck von ca. 800 mbar vollständig in Lösung. Anschliessend wird die nun klare, goldbraune Lösung 2 bis 3 Stunden bei 130°C gerührt. Der Druck steigt dabei auf ca. 1800 mbar. Die Reaktion gilt als beendet, wenn im Dünnschichtchromatogramm weniger als 2 % 2,6-Dicyclohexylphenol nachgewiesen werden.

Die nun rotbraun gewordene Lösung wird auf ca. 100°C abgekühlt und der Kolben bei Normaldruck via Kugelhahn und Brüde geöffnet. Durch Aufheizen auf 130°C und Anlegen eines Vakuums von bis zu 200 mbar wird ein Gemisch von Dimethylformamid/Wasser abdestilliert (ca. 40 g Destillat mit einem Wassergehalt von 50-60 %). Die so vom Wasser weitgehend befreite Lösung wird in einen 1500 ml Sulfierkolben gegossen. Bei einer Innentemperatur von ca. 110°C werden unter Rückfluss 200 ml Spülmethanol und anschliessend weitere 150 ml Methanol zugetropft. Die Innentemperatur sinkt dabei auf ca. 70°C ab. Die Lösung wird angeimpft. Die schnell kristallisierende Lösung wird noch ca. 1 Stunde bei 70 bis 72°C gerührt. Der gelbe Kristallbrei wird innerhalb von ca. 4 bis 5 Stunden auf Raumtemperatur und anschliessend mit Eis weiter auf 0 bis 5°C abgekühlt. Es wird noch 1 Stunde bei 0 bis 5°C gerührt und dann wird das Produkt abgenutscht. Das Nutschgut wird dreimal mit 100 ml Kaltem Methanol farblos gewaschen. Anschliessend wird es abgesaugt und bei 70 bis 80°C im Vakuumofen getrocknet.

Die Ausbeute beträgt 263 g (93 % der Theorie).

Das Produkt, welches als weisses Pulver vorliegt, besitzt einen Schmelzpunkt von 243-250°C.

Beispiel 2: Herstellung von N,N',N''-Tris[3-cyclohexyl-4-hydroxy-5-methylbenzyl]isocyanurat

In einem Sulfierkolben mit Propellerrührer, Thermometer und Rückflusskühler werden
3,23 g (25 mmol) Cyanursäure,
14,70 g (77 mmol) 2-Cyclohexyl-6-methylphenol,
2,50 g (83 mmol) Paraformaldehyd,
0,30 g (2,1 mmol) Hexamethylentetramin und
47,50 g (50 ml) Dimethylformamid
vorgelegt. Die Suspension wird in einer Stickstoffatmosphäre unter langsamem Rühren auf 110°C geheizt

und 48 Stunden am Rückfluss erhitzt. Danach wird das Reaktionsgemisch auf Raumtemperstur abgekühlt, auf 150 ml Eiswasser gegossen und gerührt. Das weisse Produkt wird abfiltriert, nach mehrmaligem Waschen mit Wasser in Diethylether gelöst und mit Wasser extrahiert. Nach Entfernung des Ethers unter vermindertem Druck erhält man das Produkt in Form eines gelblichen Schaums, der aus wässerigem Ethanol kristallisiert wird.

Die Ausbeute beträgt 14,5 g (78 % der Theorie).

Das Produkt, welches in Form von farblosen Kristallen vorliegt, besitzt einen Schmelzpunkt von 212-218°C.

Elementaranalyse:

Berechnet: N 5,71 %
Gefunden: N 5,31 %

Beispiel 3: Herstellung von N,N',N''-Tris[3-tert-butyl-5-cyclohexyl-4-hydroxybenzyl]isocyanurat

Die Herstellung erfolgt in Analogie zu Beispiel 2. Statt 2-Cyclohexyl-6-methylphenol wird die entsprechende Menge 2-tert-Butyl-6-cyclohexylphenol eingesetzt. Nach Waschen mit heissem Hexan erhält man 33,24 g (77 % der Theorie) eines weissen Pulvers mit einem Schmelzpunkt von 220°C.

Elementaranalyse:

Berechnet: N 4,87 %
Gefunden: N 5,05 %

Beispiel 4:

In der gleichen Apparatur, wie in Beispiel 2 beschrieben, werden
6,45 g (50 mmol) Cyanursäure,
10,73 g (52 mmol) 2,6-Di-tert-butylphenol,
26,70 g (103 mmol) 2,6-Dicyclohexylphenol,
16,3 g (195 mmol) Formalin-Lösung (36%ig),
0,50 g (4 mmol) Hexamethylentetramin und
71,3 g (75 ml) Dimethylformamid

vorgelegt. Die Suspension wird in einer Stickstoffatmosphäre unter langsamem Rühren auf ca. 110°C geheizt und 42 Stunden am Rückfluss erhitzt. Das auf Raumtemperatur abgekühlte Reaktionsgemisch wird auf 250 ml Wasser gegossen und gut gerührt. Das Reaktionsprodukt wird abfiltriert. Die Aufarbeitung erfolgt in Analogie zu Beispiel 3 unter Verwendung von 200 ml Petrolether (30-60°C). Man erhält ein gummiartiges Produkt und nach Umkristallisation mit 200 ml Petrolether ein pulver, das einen Schmelzpunkt von 127-131°C besitzt.

Die Ausbeute beträgt 35,4 g (80 % der Theorie).

Elementaranalyse:

Berechnet: N 4,73 %
Gefunden: N 4,51 %

Beispiel 5:

In Analogie zu Beispiel 4 werden
6,45 g (50 mmol) Cyanursäure,
21,25 g (103 mmol) 2,6-Di-tert-butylphenol,
13,44 g (52 mmol) 2,6-Dicyclohexylphenol,
16,3 g (195 mmol) Formalin-Lösung (36%ig),
0,50 g (4 mmol) Hexamethylentetramin und
71,3 g (75 ml) Dimethylformamid

umgesetzt. Die Aufarbeitung des Produkts erfolgt wie in Beispiel 4 unter Verwendung von Diethylether. Man erhält ein Pulver mit einem Schmelzpunkt von 122°C.

Die Ausbeute beträgt 23 g (54 % der Theorie).

Elementaranalyse:

Berechnet: N 5,03 %
Gefunden: N 5,13 %

Beispiel 6:

In Analogie zu Beispiel 4 werden
6,45 g (50 mmol) Cyanursäure,
10,73 g (52 mmol) 2,6-Di-tert-butylphenol,
19,60 g (103 mmol) 2-Cyclohexyl-6-methylphenol,
16,3 g (195 mmol) Formalin-Lösung (36%ig),
0,50 g (4 mmol) Hexamethylentetramin und
71,3 g (75 ml) Dimethylformamid
umgesetzt. Das Reaktionsgemisch wird abgekühlt, auf 250 ml Wasser gegossen und gut gerührt. Das Produkt wird abfiltriert und kann durch Säulenchromatographie an Kieselgel weiter gereinigt werden. Man erhält ein hellgelbes Pulver mit einem Schmelzpunkt von 114-120°C.

Die Ausbeute beträgt 22,97 g (79 % der Theorie).

Elementaranalyse:

Berechnet: N 5,49 %
Gefunden: N 5,30 %

Beispiel 7:

In Analogie zu Beispiel 4 werden
6,45 g (50 mmol) Cyanursäure,
21,25 g (103 mmol) 2,6-Di-tert-butylphenol,
9,90 g (52 mmol) 2-Cyclohexyl-6-methylphenol,
16,3 g (195 mmol) Formalin-Lösung (36%ig),
0,5 g (4 mmol) Hexamethylentetramin und
71,3 g (75 ml) Dimethylformamid
umgesetzt. Die Aufarbeitung erfolgt wie in Beispiel 3 beschrieben. Man erhält ein gummiartiges Produkt, welches nach nochmaliger Umkristallisation mit Hexan als Pulver mit einem Schmelzpunkt von 110-120°C vorliegt.

Die Ausbeute beträgt 21,60 g (56 % der Theorie).

Elementaranalyse:

Berechnet: N 5,47 %
Gefunden: N 5,75 %

Beispiel 8:

Polypropylenpulver (Schmelzindex bei 230°C und einer Prüfkraft von 2,16 kp (=21,19N):2,3 g/10 min) enthaltend 0,1 % Calciumstearat wird mit den in der nachstehenden Tabelle 1 aufgeführten Additiven gemischt und anschliessend in einem Brabender Plastographen bei 200°C 10 Minuten geknetet. Die so erhaltene Masse wird in einer Presse mit einer Oberflächentemperatur von 260°C zu 1 mm dicken Platten gepresst, aus denen Streifen von 1 cm Breite und 8,5 cm Länge gestanzt werden. Von jeder Platte werden mehrere solcher Streifen in einen auf 149°C geheizten Umluftofen gehängt und in regelmässigen Zeitabständen beobachtet. Die oxidative Zersetzung dieser Streifen lässt sich an einer kreisartig beginnenden Gelbverfärbung erkennen. Ein Mass für die Stabilität der Probe ist die Zeitdauer bis zur Zersetzung.

Tabelle 1:

| Stabilisator | Tage im Umluftofen bei 149°C bis zum Zerfall des Prüfkörpers |
|---|---|
| ohne | < 1 |
| 0,3 % DSTDP | 8 |
| 0,3 % DSTDP + 0,1 % der Verbindung aus Beispiel 1A | 91 |

DSTDP = Distearylthiodipropionat

## Patentansprüche

1. Verbindungen der Formel I,

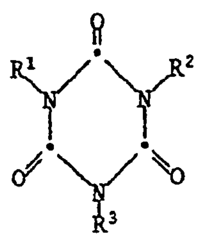

(I)

worin $R^1$ eine Gruppe der Formel II, $R^2$ eine Gruppe der Formel III und $R^3$ eine Gruppe der Formel IV bedeuten,

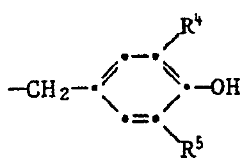

(II)

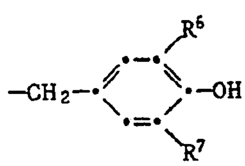

(III)

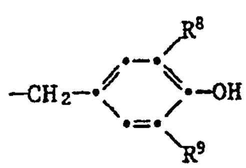

(IV)

worin $R^4$ $C_5$-$C_7$-Cycloalkyl oder durch $C_1$-$C_4$-Alkyl substituiertes $C_5$-$C_7$-Cycloalkyl ist, $R^5$, $R^6$, $R^7$, $R^8$ und $R^9$ unabhängig voneinander $C_1$-$C_{18}$-Alkyl, $C_5$-$C_7$-Cycloalkyl, durch $C_1$-$C_4$-Alkyl substituiertes $C_5$-$C_7$-Cycloalkyl, Phenyl,

Benzyl oder Allyl sind.

2. Verbindungen gemäss Anspruch 1, worin die Reste $R^5$ bis $R^9$ unabhängig voneinander $C_1$-$C_8$-Alkyl, Cyclohexyl, 1-Methylcyclohexyl, Phenyl oder Allyl bedeuten.

3. Verbindungen gemäss Anspruch 1, worin $R^4$ Cyclohexyl oder 1-Methylcyclohexyl ist.

4. Verbindungen gemäss Anspruch 1, worin $R^4$ Cyclohexyl bedeutet und die Reste $R^5$ bis $R^9$ unabhängig voneinander Methyl, tert-Butyl, Cyclohexyl oder Phenyl sind.

5. Verbindungen gemäss Anspruch 1, worin die Reste $R^1$ bis $R^3$ gleich sind.

6. Verbindungen gemäss Anspruch 5, worin $R^4$, $R^6$ und $R^8$ Cyclohexyl bedeuten und die Reste $R^5$, $R^7$ und $R^9$ gleich sind und Methyl, tert-Butyl, Cyclohexyl oder Phenyl darstellen.

7. Verbindungen gemäss Anspruch 1, worin $R^1$ verschieden von $R^2$ ist und $R^2$ und $R^3$ gleich sind.

8. Verbindungen gemäss Anspruch 1, worin $R^1$ und $R^2$ gleich sind und $R^2$ verschieden von $R^3$ ist.

9. Verbindungen gemäss Anspruch 1, worin die Reste $R^5$, $R^7$ und $R^9$ gleich sind und Methyl oder Cyclohexyl bedeuten.

10. Verbindung gemäss Anspruch 6, worin die Reste $R^4$, $R^6$ und $R^8$ Cyclohexyl und die Reste $R^5$, $R^7$ und $R^9$ Methyl bedeuten.

11. Verbindungen gemäss Anspruch 1, worin $R^4$ bis $R^9$ $C_5$-$C_7$-Cycloalkyl oder Methylcyclohexyl bedeuten.

12. Verbindung gemäss Anspruch 11, worin die Reste $R^4$ bis $R^9$ Cyclohexyl bedeuten.

13. Die Verbindungen N,N',N"-Tris[3-cyclohexyl-5-tert-butyl-4-hydroxybenzyl]isocyanurat, N,N'-Bis[3,5-dicyclohexyl-4-hydroxybenzyl]-N"-(3,5-di-tert-butyl-4-hydroxybenzyl)isocyanurat, N,N'-Bis[3,5-di-tert-butyl-4-hydroxybenzyl]-N"-(3,5-dicyclohexyl-4-hydroxybenzyl)isocyanurat, N,Né-Bis[3-cyclohexyl-5-methyl-4-hydroxybenzyl]-NÆ-(3,5-di-tert-butyl-4-hydroxybenzyl)isocyanurat und N,Né-Bis[3,5-di-tert-butyl-4-hydroxybenzyl]-NÆ-[3-cyclohexyl-5-methyl-4-hydroxybenzyl]isocyanurat gemäss Anspruch 1.

14. Gemisch enthaltend mindestens zwei Verbindungen der Formel I gemäss Anspruch 1.

15. Zusammensetzung, enthaltend organisches Material und mindestens eine Verbindung der Formel I gemäss Anspruch 1.

16. Zusammensetzung nach Anspruch 15, worin das organische Material ein synthetisches Polymer, ein natürliches oder synthetisches Elastomer oder eine natürliche oder synthetische funktionelle Flüssigkeit ist.

17. Zusammensetzung gemäss Anspruch 15, enthaltend als zusätzliche Komponente einen Thiosynergisten.

18. Verwendung von Verbindungen der Formel I gemäss Anspruch 1 zum Stabilisieren von organischem Material gegen thermischen, oxidativen und/oder aktinischen Abbau.

**Patentansprüche für folgende Vertragsstaaten: AT, ES**

1. Zusammensetzung enthaltend organisches Material und mindestens eine Verbindung der Formel I,

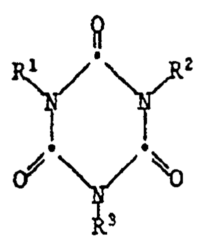

(I)

worin $R^4$ eine Gruppe der Formel II, $R^2$ eine Gruppe der Formel III und $R^3$ eine Gruppe der Formel IV bedeuten,

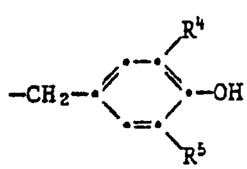

(II)

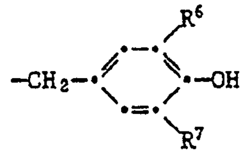

(III)

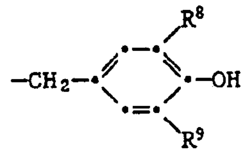

(IV)

worin $R^4$ $C_5$-$C_7$-Cycloalkyl oder durch $C_1$-$C_4$-Alkyl substituiertes $C_5$-$C_7$-Cycloalkyl ist, $R^5$, $R^6$, $R^7$, $R^8$ und $R^9$ unabhängig voneinander $C_1$-$C_{18}$-Alkyl, $C_5$-$C_7$-Cycloalkyl, durch $C_1$-$C_4$-Alkyl substituiertes $C_5$-$C_7$-Cycloalkyl, Phenyl, Benzyl oder Allyl sind.

2. Zusammensetzung gemäss Anspruch 1, worin die Reste $R^5$ bis $R^9$ unabhängig voneinander $C_1$-$C_8$-Allyl, Cyclohexyl, 1-Methylcyclohexyl, Phenyl oder Allyl bedeuten.

3. Zusammensetzung gemäss Anspruch 1, worin $R^4$ Cyclohexyl oder 1-Methylcyclohexyl ist.

4. Zusammensetzung gemäss Anspruch 1, worin $R^4$ Cyclohexyl bedeutet und die Reste $R^5$ bis $R^9$ unabhängig voneinander Methyl, tert-Butyl, Cyclohexyl oder Phenyl sind.

5. Zusammensetzung gemäss Anspruch 1, worin die Reste $R^1$ bis $R^3$ gleich sind.

6. Zusammensetzung gemäss Anspruch 5, worin $R^4$, $R^6$ und $R^8$ Cyclohexyl bedeuten und die Reste $R^5$, $R^7$ und $R^9$ gleich sind und Methyl, tert-Butyl, Cyclohexyl oder Phenyl darstellen.

7. Zusammensetzung gemäss Anspruch 1, worin $R^1$ verschieden von $R^2$ ist und $R^2$ und $R^3$ gleich sind.

8. Zusammensetzung gemäss Anspruch 1, worin $R^1$ und $R^2$ gleich sind und $R^2$ verschieden von $R^3$ ist.

9. Zusammensetzung gemäss Anspruch 1, worin die Reste $R^5$, $R^7$ und $R^9$ gleich sind und Methyl oder Cyclohexyl bedeuten.

10. Zusammensetzung gemäss Anspruch 6, worin die Reste $R^4$, $R^6$ und $R^8$ Cyclohexyl und die Reste $R^5$, $R^7$ und $R^9$ Methyl bedeuten.

11. Zusammensetzung gemäss Anspruch 1, worin $R^4$ bis $R^9$ $C_5$-$C_7$-Cycloalkyl oder Methylcyclohexyl bedeuten.

12. Zusammensetzung gemäss Anspruch 11, worin die Reste $R^4$ bis $R^9$ Cyclohexyl bedeuten.

13. Zusammensetzung gemäss Anspruch 1, worin die Verbindung der Formel I N,N',N''-Tris[3-cyclohexyl-5-tert-butyl-4-hydroxybenzyl]isocyanurat, N,N'-Bis[3,5-dicyclohexyl-4-hydroxybenzyl]-N''-(3,5-di-tert-butyl-4-hydroxybenzyl)isocyanurat, N,N'-Bis[3,5-di-tert-butyl-4-hydroxybenzyl]-N''-(3,5-dicyclohexyl-4-hydroxybenzyl)isocyanurat, N,N'-Bis[3-cyclohexyl-5-methyl-4-hydroxybenzyl]-N''-(3,5-di-tert-butyl-4-hydroxybenzyl)isocyanurat oder N,N'-Bis[3,5-di-tert-butyl-4-hydroxybenzyl]-N''-[3-cyclohexyl-5-methyl-4-hydroxybenzyl]isocyanurat ist.

14. Zusammensetzung gemäss Anspruch 1, worin das organische Material ein synthetisches Polymer, ein natürliches oder synthetisches Elastomer oder eine natürliche oder synthetische funktionelle Flüssigkeit ist.

15. Zusammensetzung gemäss Anspruch 1, enthaltend als zusätzliche Komponente einen Thiosynergisten.

16. Verfahren zum Stabilisieren von organischem Material gegen thermischen, oxidativen und/oder aktinischen Abbau, dadurch gekennzeichnet, dass man dem organischen Material mindestens eine Verbindung der Formel I nach Anspruch 1 einverleibt.

17. Verfahren zur Herstellung von Verbindungen der Formel I nach Anspruch 1, dadurch gekennzeichnet, dass man ein Phenol der Formel V oder Mischungen von Phenolen der Formeln V, VI und VII,

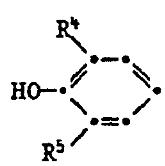

(V),

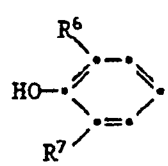

(VI),

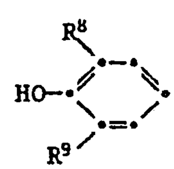

(VII)

worin die Reste $R^4$ bis $R^9$ die in Anspruch 1 angegebenen Bedeutungen besitzen, mit Isocyanursäure und Formaldehyd oder einer Formaldehyd freisetzenden Verbindung, gegebenenfalls in einem Lösungsmittel, umsetzt.

**Claims**

1. A compound of the formula I

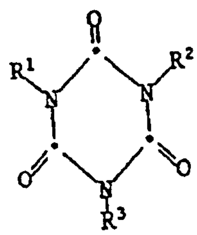

(I)

in which $R^1$ is a group of the formula II, $R^2$ is a group of the formula III and $R^3$ is a group of the formula IV

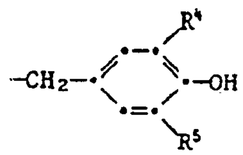

(II)

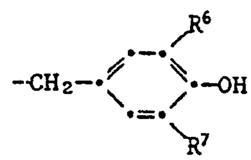

(III)

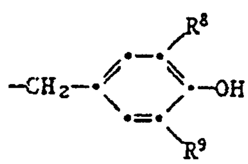

(IV)

in which $R^4$ is a $C_5$-$C_4$cycloalkyl or $C_5$-$C_7$cycloalkyl substituted by $C_1$-$C_4$alkyl, $R^5$, $R^6$, $R^7$, $R^8$ and $R^9$ are independently of one another $C_1$-$C_{18}$ alkyl, $C_5$-$C_7$cycloalkyl, $C_5$-$C_7$cycloalkyl substituted by $C_1$-$C_4$alkyl, phenyl, benzyl or allyl.

2. A compound according to claim 1, in which the radicals $R^5$ to $R^9$ are independently of one another $C_1$-$C_8$ alkyl, cyclohexyl, 1-methylcyclohexyl, phenyl or allyl.

3. A compound according to claim 1, in which $R^4$ is cyclohexyl or 1-methylcyclohexyl.

4. A compound according to claim 1, in which $R^4$ is cyclohexyl and the radicals $R^5$ to $R^9$ are independently of one another methyl, tert-butyl, cyclohexyl or phenyl.

5. A compound according to claim 1, in which the radicals $R^1$ to $R^3$ are identical.

6. A compound according to claim 5, in which $R^4$, $R^6$ and $R^8$ are cyclohexyl and the radicals $R^5$, $R^7$ and $R^9$ are identical and are methyl, tert-butyl, cyclohexyl or phenyl.

7. A compound according to claim 1, in which $R^1$ is different from $R^2$ and $R^2$ and $R^3$ are identical.

8. A compound according to claim 1, in which $R^1$ and $R^2$ are identical and $R^2$ is different from $R^3$.

9. A compound according to claim 1, in which the radicals $R^5$, $R^7$ and $R^9$ are identical and are methyl or cyclohexyl.

10. A compound according to claim 6, in which the radicals $R^4$, $R^6$ and $R^8$ are cyclohexyl and the radicals $R^5$, $R^7$ and $R^9$ are methyl.

11. A compound according to claim 1, in which $R^4$ to $R^9$ are $C_5$-$C_7$-cycloalkyl or methylcyclohexyl.

12. A compound according to claim 11, in which the radicals $R^4$ to $R^9$ are cyclohexyl.

13. The compounds N,N',N''-tris[3-cyclohexyl-5-tert-butyl-4-hydroxybenzyl] isocyanurate, N,N'-bis[3,5-dicyclohexyl-4-hydroxybenzyl]-N''-(3,5-di-tert-butyl-4-hydroxybenzyl) isocyanurate, N,N'-bis[3,5-di-tert-butyl-4-hydroxybenzyl]-N''-(3,5-dicyclohexyl-4-hydroxybenzyl) isocyanurate, N,N'-bis[3-cyclohexyl-5-methyl-4- hydroxybenzyl]-N''-(3,5-di-tert-butyl-4-hydroxybenzyl) isocyanurate and N,N'-bis[3,5-di-tert-butyl-4-hydroxybenzyl]-N''-[3-cyclohexyl-5-methyl-4-hydroxybenzyl] isocyanurate according to claim 1.

14. A mixture containing at least two compounds of the formula I according to claim 1.

15. A composition containing organic material and at least one compound of the formula I according to claim 1.

16. A composition according to claim 15, in which the organic material is a synthetic polymer, a natural or synthetic elastomer or a natural or synthetic functional fluid.

17. A composition according to claim 15, containing a thiosynergist as additional component.

18. The use of a compound of the formula I according to claim 1 for stabilising organic material against thermal, oxidative and/or actinic degradation.

**Claims for the following Contracting States: AT, ES**

1. A composition containing organic material and at least one compound of the formula I

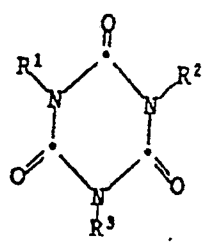

(I)

in which $R^1$ is a group of the formula II, $R^2$ is a group of the formula III and $R^3$ is a group of the formula IV

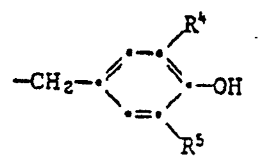

(II)

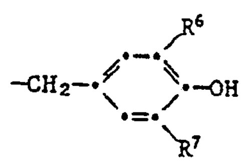

(III)

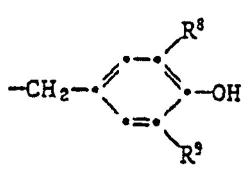

(IV)

in which $R^4$ is $C_5$-$C_7$cycloalkyl or $C_5$-$C_7$cycloalkyl substituted by $C_1$-$C_4$alkyl, $R^5$, $R^6$, $R^7$, $R^8$ and $R^9$ are independently of one another $C_1$-$C_{18}$ alkyl, $C_5$-$C_7$cycloalkyl, $C_5$-$C_7$cycloalkyl substituted by $C_1$-$C_4$alkyl, phenyl, benzyl or allyl.

2. A composition according to claim 1, in which the radicals $R^5$ to $R^9$ are independently of one another $C_1$-$C_8$ alkyl, cyclohexyl, 1-methylcyclohexyl, phenyl or allyl.

3. A composition according to claim 1, in which $R^4$ is cyclohexyl or 1-methylcyclohexyl.

4. A composition according to claim 1, in which $R^4$ is cyclohexyl and the radicals $R^5$ to $R^9$ are independently of one another methyl, tert-butyl, cyclohexyl or phenyl.

5. A composition according to claim 1, in which the radicals $R^1$ to $R^3$ are identical.

6. A composition according to claim 5, in which $R^4$, $R^6$ and $R^8$ are cyclohexyl and the radicals $R^5$, $R^7$ and $R^9$ are identical and are methyl, tert-butyl, cyclohexyl or phenyl.

7. A composition according to claim 1, in which $R^1$ is different from $R^2$ and $R^2$ and $R^3$ are identical.

8. A composition according to claim 1, in which $R^1$ and $R^2$ are identical and $R^2$ is different from $R^3$.

9. A composition according to claim 1, in which the radicals $R^5$, $R^7$ and $R^9$ are identical and are methyl or cyclohexyl.

10. A composition according to claim 6, in which the radicals $R^4$, $R^6$ and $R^8$ are cyclohexyl and the radicals $R^5$, $R^7$ and $R^9$ are methyl.

11. A composition according to claim 1, in which $R^4$ to $R^9$ are $C_5$-$C_7$-cycloalkyl or methylcyclohexyl.

12. A composition according to claim 11, in which the radicals $R^4$ to $R^9$ are cyclohexyl.

13. A composition according to claim 1, in which the compound of the formula I is N,N',N"-tris[3-cyclohexyl-5-tert-butyl-4-hydroxybenzyl] isocyanurate, N,N'-bis[3,5-dicyclohexyl-4-hydroxybenzyl]-N"-(3,5-di-tert-butyl-4-hydroxybenzyl) isocyanurate, N,N'-bis[3,5-di-tert- butyl- 4- hydroxybenzyl]- N"-(3,5- dicyclohexyl- 4- hydroxybenzyl) isocyanurate, N,N'-bis[3-cyclohexyl-5-methyl-4-hydroxybenzyl]-N"-(3,5-di-tert-butyl-4-hydroxybenzyl) isocyanurate or N,N'-bis[3,5-di-tert-butyl-4-hydroxybenzyl]-N"-[3-cyclohexyl-5-methyl-4-hydroxybenzyl] isocyanurate.

14. A composition according to claim 1, in which the organic material is a synthetic polymer, a natural or synthetic elastomer or a natural or synthetic functional fluid.

15. A composition according to claim 1, containing a thiosynergist as additional component.

16. A process for stabilising organic material against thermal, oxidative and/or actinic degradation, which comprises incorporating in the organic material at least one compound of the formula I according to claim 1.

17. A process for the preparation of a compound of the formula I according to claim 1, which comprises reacting a phenol of the formula V or a mixture of phenols of the formulae V, VI and VII

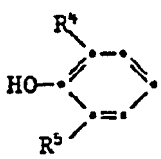 (V), 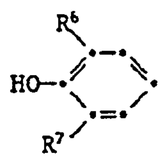 (VI), 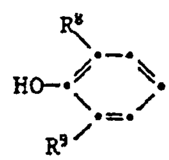 (VII)

in which the radicals $R^4$ to $R^9$ are as defined in claim 1, with isocyanuric acid and formaldehyde or a compound which liberates formaldehyde, optionally in a solvent.

## Revendications

1. Composés répondant à la formule I :

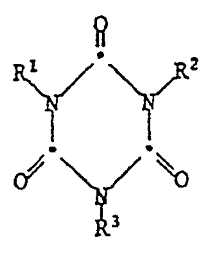

(I)

dans laquelle $R^1$ représente un radical de formule II, $R^2$ un radical de formule III et $R^3$ un radical de formule IV :

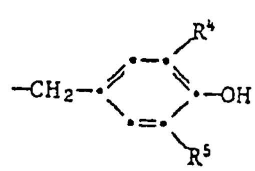

(II)

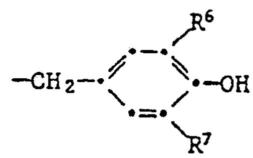

(III)

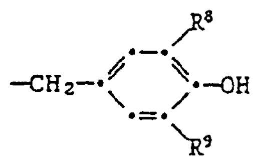

(IV),

formules dans lesquelles $R^4$ représente un cycloalkyle en $C_5$-$C_7$ éventuellement porteur d'un alkyle en $C_1$-$C_4$, et $R^5$, $R^6$, $R^7$, $R^8$ et $R^9$ représentent chacun, indépendamment les uns des autres, un alkyle en $C_1$-$C_{18}$, un cycloalkyle en $C_5$-$C_7$ éventuellement porteur d'un alkyle en $C_1$-$C_4$, un phényle, un benzyle ou un allyle.

2. Composés selon la revendication 1 dans lesquels $R^5$ à $R^9$ représentent chacun, indépendamment les uns des autres, un alkyle en $C_1$-$C_8$, un cyclohexyle, un méthyl-1 cyclohexyle, un phényle ou un allyle.

3. Composés selon la revendication 1 dans lesquels $R^4$ représente un cyclohexyle ou un méthyl-1 cyclohexyle.

4. Composés selon la revendication 1 dans lesquels $R^4$ représente un cyclohexyle et $R^5$ à $R^9$ représentent chacun, indépendamment les uns des autres, un radical méthyle, tert-butyle, cyclohexyle ou phényle.

5. Composés selon la revendication 1 dans lesquels les radicaux $R^1$ à $R^3$ sont identiques.

6. Composés selon la revendication 5 dans lesquels $R^4$, $R^6$ et $R^9$ représentent chacun un cyclohexyle et les radicaux $R^5$, $R^7$ et $R^9$ sont identiques et sont chacun un radical méthyle, tert-butyle, cyclohexyle ou phényle.

7. Composés selon la revendication 1 dans lesquels $R^1$ est différent de $R^2$, et $R^2$ et $R^3$ sont identiques.

8. Composés selon la revendication 1 dans lesquels $R^1$ et $R^2$ sont identiques, et $R^2$ est différent de $R^3$.

9. Composés selon la revendication 1 dans lesquels les radicaux $R^5$, $R^7$ et $R^9$ sont identiques et sont chacun un radical méthyle ou cyclohexyle.

10. Composé selon la revendication 1 dans lequel $R^4$, $R^6$ et $R^9$ représentent chacun un cyclohexyle, et $R^5$, $R^7$ et $R^9$ chacun un méthyle.

11. Composés selon la revendication 1 dans lesquels $R^4$ à $R^9$ représentent chacun un cycloalkyle en $C_5$-$C_7$ ou un méthylcyclohexyle.

12. Composé selon la revendication 11 dans lequel $R^4$ à $R^9$ représentent chacun un cyclohexyle.

13. Composés selon la revendication 1, en l'espèce l'isocyanurate de N,N',N"-tris-(cyclohexyl-3 tert-butyl-5-hydroxy-4 benzyle), l'isocyanurate de N,N'-bis-(dicyclohexyl-3,5 hydroxy-4 benzyle) et de N"-(di-tert-butyl-3,5 hydroxy-4 benzyle), l'isocyanurate de N,N'-bis(di-tert)butyl-3,5 hydroxy-4 benzyle) et de N"-(dicyclohexyl-3,5 hydroxy-4 benzyle), l'isocyanurate de N,N'-bis-(cyclohexyl-3 méthyl-5 hydroxy-4 benzyle) et de N"-(ditert-butyl-3,5 hydroxy-4 benzyle) et l'isocyanurate de N,N'-bis-(ditert-butyl-3,5 hydroxy-4 benzyle) et de N"-(cyclohexyl-3 méthyl-5 hydroxy-4 benzyle).

14. Mélange contenant au moins deux composés de formule I selon la revendication 1.

15. Composition contenant une matière organique et au moins un composé de formule I selon la revendication 1.

16. Composition selon la revendication 15 dans laquelle la matière organique est un polymère synthétique, un élastomère naturel ou synthétique ou un liquide fonctionnel naturel ou synthétique.

17. Composition selon la revendication 15 qui contient, comme composante supplémentaire, un agent de synergie sulfuré.

18. Application de composés de formule I selon la revendication 1 à la stabilisation de matières organiques contre la dégradation due à la chaleur, à l'oxydation et/ou à des rayonnements actiniques.

**Revendications pour les Etats Contractants: AT, ES**

1. Composition contenant une matière organique et au moins un composé répondant à la formule I :

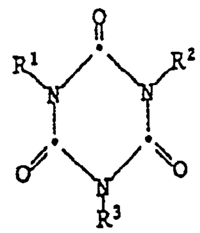

(I)

dans laquelle $R^1$ représente un radical de formule II, $R^2$ un radical de formule III et $R^3$ un radical de formule IV :

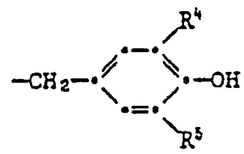

(II)

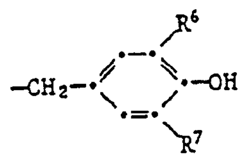

(III)

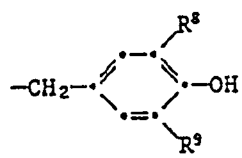

(IV),

formules dans lesquelles $R^4$ représente un cycloalkyle en $C_5$-$C_7$ éventuellement porteur d'un alkyle en $C_1$-$C_4$, et $R^5$, $R^6$, $R^7$, $R^8$ et $R^9$ représentent chacun, indépendamment les uns des autres, un alkyle en $C_1$-$C_{18}$, un cycloalkyle en $C_5$-$C_7$ éventuellement porteur d'un alkyle en $C_1$-$C_4$, un phényle, un benzyle ou un allyle.

2. Composition selon la revendication 1 dans laquelle $R^5$ à $R^9$ représentent chacun, indépendamment les uns des autres, un alkyle en $C_1$-$C_8$, un cyclohexyle, un méthyl-1-cyclohexyle, un phényle ou un allyle.

3. Composition selon la revendication 1 dans laquelle $R^4$ représente un cyclohexyle ou un méthyl-1-cyclohexyle.

4. Composition selon la revendication 1 dans laquelle $R^4$ représente un cyclohexyle et $R^5$ à $R^9$ représentent chacun, indépendamment les uns des autres, un radical méthyle, tert-butyle, cyclohexyle ou phényle.

5. Composition selon la revendication 1 dans laquelle les radicaux $R^1$ à $R^3$ sont identiques.

6. Composition selon la revendication 5 dans laquelle $R^4$, $R^6$ et $R^8$ représentent chacun un cyclohexyle et les radicaux $R^5$, $R^7$ et $R^9$ sont identiques et sont chacun un radical méthyle, tert-butyle, cyclohexyle ou phényle.

7. Composition selon la revendication 1 dans laquelle $R^1$ est différent de $R^2$, et $R^2$ et $R^3$ sont identiques.

8. Composition selon la revendication 1 dans laquelle $R^1$ et $R^2$ sont identiques, et $R^2$ est différent de $R^3$.

9. Composition selon la revendication 1 dans laquelle les radicaux $R^5$, $R^7$ et $R^9$ sont identiques et sont chacun un radical méthyle ou cyclohexyle.

10. Composition selon la revendication 6 dans laquelle $R^4$, $R^6$ et $R^8$ représentent chacun un cyclohexyle, et $R^5$, $R^7$ et $R^9$ chacun un méthyle.

11. Composition selon la revendication 1 dans laquelle $R^4$ à $R^9$ représentent chacun un cycloalkyle en $C_5$-$C_7$ ou un méthylcyclohexyle.

12. Composition selon la revendication 11 dans laquelle $R^4$ à $R^9$ représentent chacun un cyclohexyle.

13. Composition selon la revendication 1, dans laquelle le composé de formule I est l'isocyanurate de N,N',N"-tris-(cyclohexyl-3 tert-butyl-5 hydroxy-4 benzyle), l'isocyanurate de N,N'-bis-(dicyclohexyl-3,5 hydroxy-4-benzyle) et de N"-(di-tert-butyl-3,5 hydroxy-4 benzyle), l'isocyanurate de N,N'-bis-(di-tert)butyl-3,5 hydroxy-4-benzyle) et de N"-(dicyclo-hexyl-3,5 hydroxy-4 benzyle), l'isocyanurate de N,N'-bis-(cyclohexyl-3

méthyl-5 hydroxy-4 benzyle) ou de N''-(ditert-butyl-3,5 hydroxy-4 benzyle) ou l'isocyanurate de N,N'-bis-(ditert-butyl-3,5 hydroxy-4 benzyle) et de N''-(cyclohexyl-3 méthyl-5 hydroxy-4 benzyle).

14. Composition selon la revendication 1 dans laquelle la matière organique est un polymère synthétique, un élastomère naturel ou synthétique ou un liquide fonctionnel naturel ou synthétique.

15. Composition selon la revendication 1 qui contient, comme composante supplémentaire, un agent de synergie sulfuré.

16. Procédé pour stabiliser une matière organique contre contre la dégradation due à la chaleur, à l'oxydation et/ou à des rayonnements actiniques, procédé caractérisé en ce qu'on incorpore à la matière organique au moins un composé de formule I selon la revendication 1.

17. Procédé pour préparer des composés de formule I selon la revendication 1, caractérisé en ce qu'on fait réagir un phénol de formule V ou des mélanges de phénols répondant aux formules V, VI et VII :

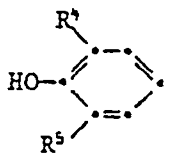 (V), 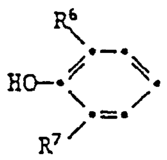 (VI), 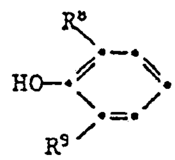 (VII)

dans lesquelles les symboles $R^4$ à $R^9$ ont les significations qui leur ont été données à la revendication 1, avec l'acide isocyanurique et le formaldéhyde ou un composé libérant du formaldéhyde, éventuellement dans un solvant.